# EUROPEAN PATENT APPLICATION

(11) **EP 4 365 213 A1**
(43) Date of publication of application: **08.05.2024**
(21) Application number: 22832920.7
(22) Date of filing: 21.06.2022
(51) Int. Cl.: C08F 212/00, C08F 220/00, C08F 226/02, G01N 27/327

(54) **POLYMER, REAGENT LAYER, AND SENSOR**

(30) Priority: 29.06.2021 JP 2021107307
(71) Applicant: PHC Holdings Corporation, Tokyo 105-8433 (JP)
(72) Inventor: HANEDA, Keigo, Ehime 7910395 (JP)
(74) Representative: Eisenführ Speiser
(86) International application number: PCT/JP2022/024652
(87) International publication number: WO 2023/276772

(57) **Abstract**

A polymer (36) included in a reagent layer (32) of a sensor (1) for measuring an analyte in a sample includes: a first binding site (46) covalently bonded to a protein (38) acting on the analyte; and a second binding site (48) that binds to the protein (38) by electrostatic interaction.

## Description

### [TECHNICAL FIELD]

The present disclosure relates to polymers, reagent layers and sensors.

### [BACKGROUND ART]

Conventionally, a sensor is known that measures an analyte in a sample by causing a protein to act on the analyte. Such sensors include electrochemical sensors using an enzyme. A representative example of the electrochemical sensor using an enzyme is an electrochemical glucose biosensor used for self-measurement of blood glucose. In the electrochemical glucose biosensor, glucose oxidoreductase is used.

In recent years, implantable electrochemical sensors have been developed that continuously measure an analyte in vivo. Such implantable electrochemical sensors are generally implanted in a living body for a long period of time such as several days to several weeks. It is therefore necessary to inhibit the outflow of oxidoreductase outside the sensor. Meanwhile, a technique is known that inhibits the outflow of oxidoreductase outside the sensor by covering the reagent layer including the oxidoreductase with a protective film (see, for example, patent literature 1).

[Patent literature 1] WO2019/146788

### [SUMMARY OF INVENTION]

### [TECHNICAL PROBLEM]

By inhibiting the outflow of oxidoreductase by using the protective film, it is possible to inhibit a decrease in the durability and measurement sensitivity of the electrochemical sensor and negative impacts on the living body, etc. Meanwhile, there is always a demand to further suppress a decrease in the durability and measurement sensitivity of the electrochemical sensor and to further inhibit negative impacts on the living body, etc. Furthermore, maintenance of durability and measurement sensitivity is naturally required even in non-implantable sensors and sensors that use proteins other than oxidoreductase. It is therefore desired to further suppress the outflow of protein outside the sensor.

The present disclosure addresses the issue described above, and a purpose thereof is to provide a technique to inhibit the outflow of the protein outside the sensor.

### [SOLUTION TO PROBLEM]

An embodiment of the present disclosure relates to a polymer included in a reagent layer of a sensor for measuring an analyte in a sample. The polymer includes a first binding site covalently bonded to a protein acting on the analyte; and a second binding site that binds to the protein by electrostatic interaction.

Another embodiment of the present disclosure relates to a reagent layer of a sensor for measuring an analyte in a sample. The reagent layer includes the polymer according to the above embodiment; and the protein that binds to the polymer and also acts on the analyte.

Another embodiment of the present disclosure relates to a sensor. The sensor includes an electrode part that includes a working electrode and a counter electrode; and the reagent layer according to the above embodiment arranged so as to be in contact with the working electrode.

Optional combinations of the constituting elements described above, and implementations of the disclosure in the form of methods, apparatuses, systems, etc. may also be practiced as additional embodiments of the present disclosure.

### [ADVANTAGEOUS EFFECTS OF INVENTION]

According to this disclosure, it is possible to inhibit the outflow of the protein outside the sensor.

### [BRIEF DESCRIPTION OF DRAWINGS]

Fig. 1A is a diagram showing the front surface side of the sensor according to the embodiment;
Fig. 1B is a diagram showing the back surface side of the sensor according to the embodiment;
Figs. 2A, 2B, 2C and 2D are diagrams showing steps of manufacturing the sensor;
Figs. 3A, 3B, 3C and 3D are diagrams showing steps of manufacturing the sensor;
Figs. 4A, 4B and 4C are diagrams showing steps of manufacturing the sensor;
Fig. 5 is a cross-sectional view along the A-A line shown in Fig. 1A;
Fig. 6A is a cross-sectional view along the B-B line shown in Fig. 5;
Fig. 6B is a cross-sectional view along the C-C line shown in Fig. 5;
Fig. 7 is a schematic diagram showing the state of a polymer, a protein, and a redox mediator in the reagent layer;
Fig. 8 is a diagram showing combinations of reactive groups that can be covalently bonded;
Fig. 9 is a schematic diagram for explaining the measurement principle of the sensor;
Fig. 10 is a diagram showing the polymerization reaction of polymer A;
Fig. 11 is a diagram showing the polymerization reaction of polymer B;
Fig. 12 is a diagram showing the polymerization reaction of polymer C;
Fig. 13 is a diagram showing the absorption spectrum of each mediator-bonded polymer;
Fig. 14 is a diagram illustrating the results of durability evaluation of the sensor;
Fig. 15 is a diagram showing the polymerization reaction of polymer E; and
Fig. 16 is a diagram showing the polymerization reaction of polymer F.

### [DESCRIPTION OF EMBODIMENTS]

Hereinafter, the present disclosure will be described based on preferred embodiments with reference to drawings. The embodiments do not limit the scope of the present disclosure but exemplify the disclosure. Not all of the features and the combinations thereof described in the embodiments are necessarily essential to the present disclosure. Identical or like constituting elements, members, processes shown in the drawings are represented by identical symbols and a duplicate description will be omitted as appropriate. The scales and shapes shown in the figures are defined for convenience's sake to make the explanation easy and shall not be interpreted limitatively unless otherwise specified. Terms like "first", "second", etc. used in the specification and claims do not indicate an order or importance by any means unless otherwise specified and are used to distinguish a certain feature from the others. Those of the members that are not important in describing the embodiment are omitted from the drawings.

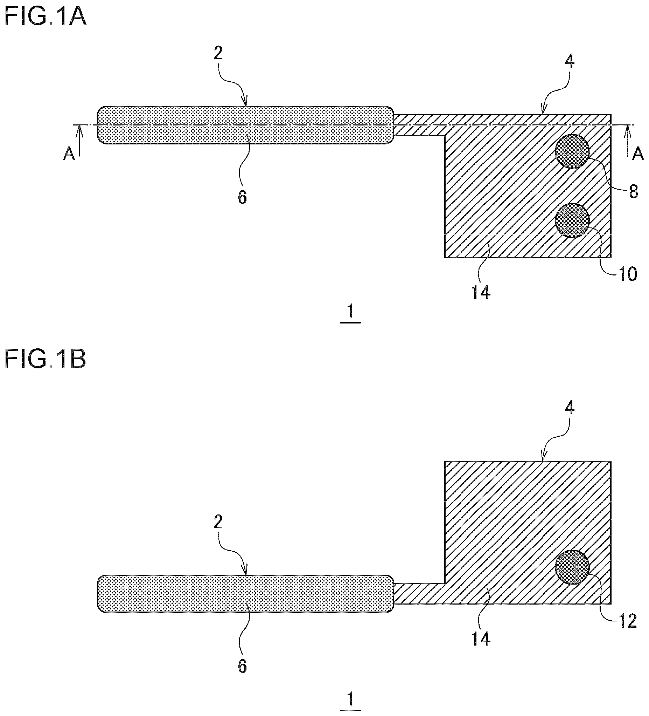

Fig. 1A is a diagram showing the front surface side of the sensor 1 according to the embodiment. Fig. 1B is a diagram showing the back surface side of the sensor 1 according to the embodiment. In this embodiment, the front surface of the sensor 1 is a surface on the side where a working electrode 26 and a reference electrode 28 are arranged. The back surface of the sensor 1 is a surface on the side where a counter electrode 30 is arranged. The front and back of the sensor 1 are not particularly limited.

A sensor 1 according to this embodiment is a sensor for measuring an analyte in a sample. For example, the sensor 1 is a biosensor. The sensor 1 is exemplified by an electrochemical sensor and includes a sensing unit 2 and a terminal unit 4. The sensing unit 2 is inserted into a living body (for example, a human body). The terminal unit 4 electrically connects a circuit unit (not shown) attached to the surface of the living body and the sensing unit 2. The sensing unit 2 transmits an electrical signal to the circuit unit via the terminal unit 4.

By maintaining a state in which the sensing unit 2 is inserted into the living body, the presence and the concentration of glucose or the like as an analyte in the blood, etc. as a sample can be continuously measured. In this embodiment, an implantable electrochemical sensor will be described as an example of the sensor 1, but the sensor 1 is optionally a non-implantable type, or a measurement mode other than electrochemical is optionally adopted. Further, the type of sample and analyte is not particularly limited. The sample may be, for example, a medium or the like. The analyte is exemplified by hemoglobin; glycated hemoglobin; amino acids such as glutamine, glutamate; glycated amino acids such as fructosyllysine, fructosylvaline; glycated peptides such as fructosylvalylhistidine; glycated albumin; sugars such as glucose; vitamins such as vitamin C; alcohols; cholesterol; lactic acid; pyruvic acid; ketone bodies (3-hydroxybutyric acid), etc.

The sensing unit 2 is covered with a protective film 6. The protective film 6 covers at least the reagent layer described later. The protective film 6 inhibits leakage of substances included in the reagent layer (for example, oxidoreductase and redox mediator) outside the protective film 6. Further, the protective film 6 has pores through which an analyte existing outside the protective film 6 can permeate into the protective film 6. In the implantable biosensor, the sensing unit 2 is inserted into a living body. Preferably, therefore, proteins and cells are not easily adsorbed to the protective film 6. Generally, the protective film 6 is made of a polymer having such a property. Examples of the polymer that can form the protective film 6 include a copolymer of methyl methacrylate and hydroxyethyl methacrylate, a copolymer of butyl methacrylate and hydroxyethyl methacrylate, and poly (2-methacryloyloxyethylphosphorylcholine-co-n-butyl methacrylate). (Meth) acrylate compounds having a backbone similar to these exemplary polymers can also be used.

A reference electrode connection terminal 8 and a working electrode connection terminal 10 are provided on the front surface of the terminal unit 4. A counter electrode connection terminal 12 is provided on the back surface of the terminal unit 4. The portions of the terminal unit 4 excluding the reference electrode connection terminal 8, the working electrode connection terminal 10, and the counter electrode connection terminal 12 are covered with an insulating resist film 14.

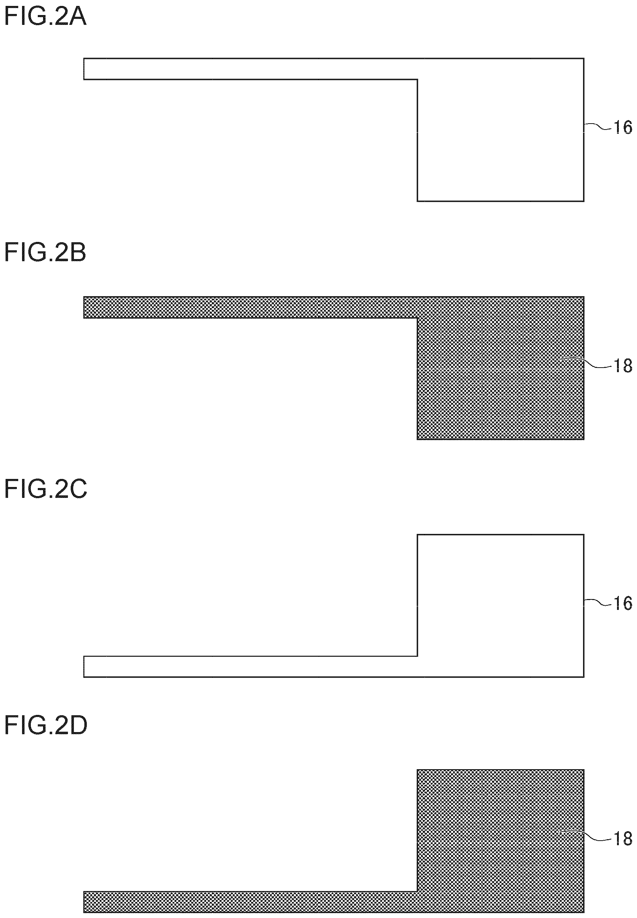

Figs. 2A to 2D, 3A to 3D, and 4A to 4C are diagrams showing steps of manufacturing the sensor 1. First, as shown in Figs. 2A and 2C, an insulating substrate 16 is formed. The insulating substrate 16 is made of, for example, a resin material such as polyethylene terephthalate, polyetherketone, polycarbonate, polyimide, polyethylene, polypropylene, polystyrene, polyvinyl chloride, polyoxymethylene, monomercast nylon, polybutylene terephthalate, methacrylate resin, ABS resin, or is made of a glass material. Preferably, the insulating substrate 16 is made of polyethylene terephthalate.

Then, as shown in Fig. 2B, a conductive thin film 18 is formed on the front surface of the insulating substrate 16. Further, as shown in Fig. 2D, a conductive thin film 18 is formed on the back surface of the insulating substrate 16. The conductive thin film 18 can be formed by depositing a conductive material such as gold (Au), platinum (Pt), palladium (Pd), iridium (Ir), and carbon (C) on both surfaces of the insulating substrate 16 by using a sputtering method, a vapor deposition method, an ion plating method, a screen-printing method, or the like.

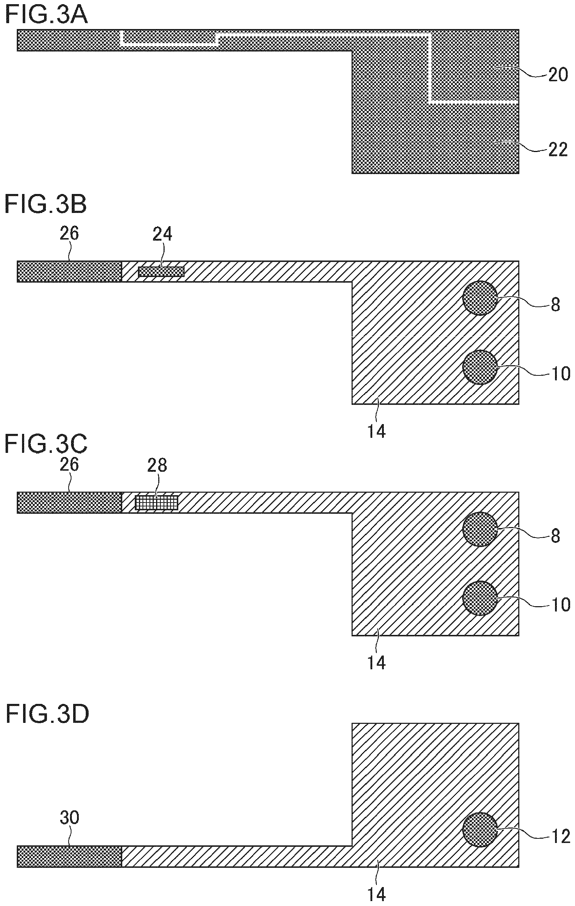

Next, as shown in Fig. 3A, the conductive thin film 18 on the front surface side is divided by laser drawing to form a reference electrode conductor 20 and a working electrode conductor 22. Then, as shown in Fig. 3B, a resist film 14 is formed on the conductive thin film 18 on the front side by a screen-printing method or the like. The resist film 14 is not stacked on a portion of the reference electrode conductor 20 included in the sensing unit 2 and a portion of the reference electrode conductor 20 included in the terminal unit 4 so that these portions are exposed. Thereby, a reference electrode formation region 24 and the reference electrode connection terminal 8 are formed. Further, the resist film 14 is not stacked on a portion of the working electrode conductor 22 included in the sensing unit 2 and a portion of the working electrode conductor 22 included in the terminal unit 4 so that these parts are exposed. Thereby, the working electrode 26 and the working electrode connection terminal 10 are formed. Next, as shown in Fig. 3C, silver/silver chloride (Ag/AgCl) or the like is deposited in the reference electrode formation region 24 by a screen-printing method, an inkjet printing method, or the like to form the reference electrode 28.

Further, as shown in Fig. 3D, a resist film 14 is formed on the conductive thin film 18 on the back surface side by a screen-printing method or the like. The resist film 14 is not stacked on a portion of the conductive thin film 18 on the back side included in the sensing unit 2 and a portion of the conductive thin film 18 on the back side included in the terminal unit 4 so that these portions are exposed. Thereby, the counter electrode 30 and the counter electrode connection terminal 12 are formed.

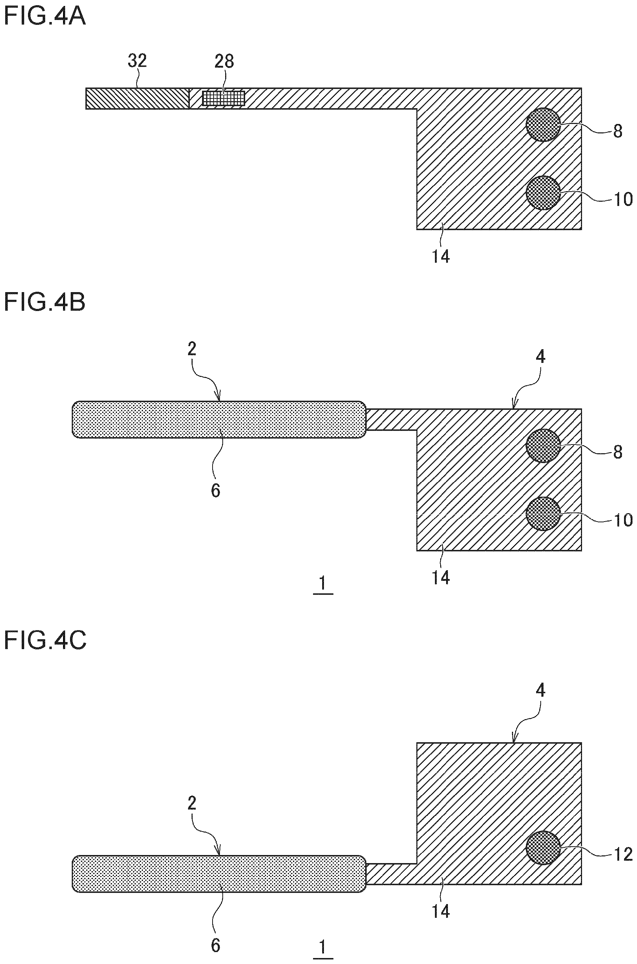

Next, as shown in Fig. 4A, a reagent solution is applied on the working electrode 26 and is dried to form a reagent layer 32. Therefore, the reagent layer 32 is arranged so as to be in contact with the working electrode 26. The composition of the reagent layer 32 will be described in detail later. Subsequently, as shown in Figs. 4B and 4C, the portion corresponding to the sensing unit 2 is immersed in a protective film polymer solution and is dried to form the protective film 6. Thereby, the reagent layer 32 is covered with the protective film 6. The protective film 6 of this embodiment covers the entire sensing unit 2.

Through the above steps, the sensor 1 having the sensing unit 2 covered with the protective film 6 and having the terminal unit 4, which is not covered with the protective film 6 and in which the reference electrode connection terminal 8, the working electrode connection terminal 10, and the counter electrode connection terminal 12 are exposed, is obtained.

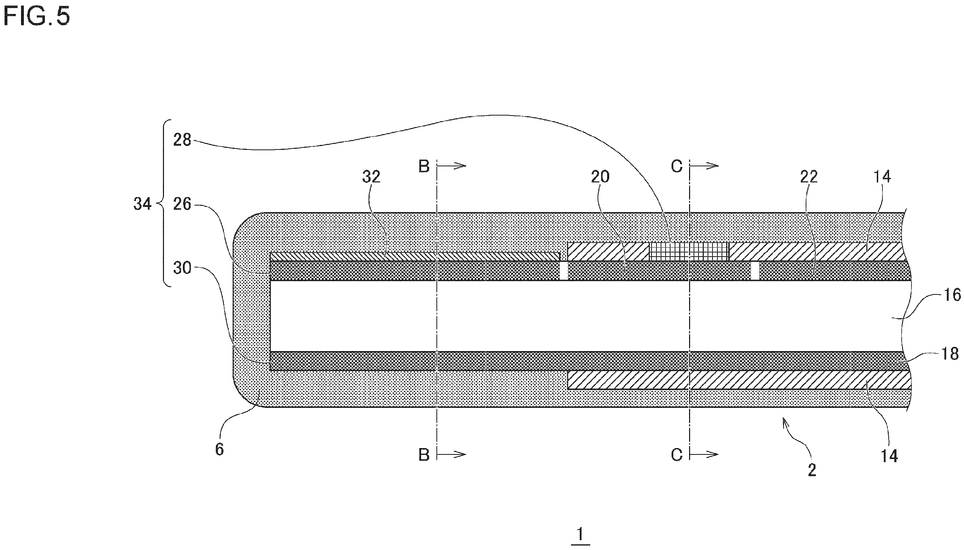

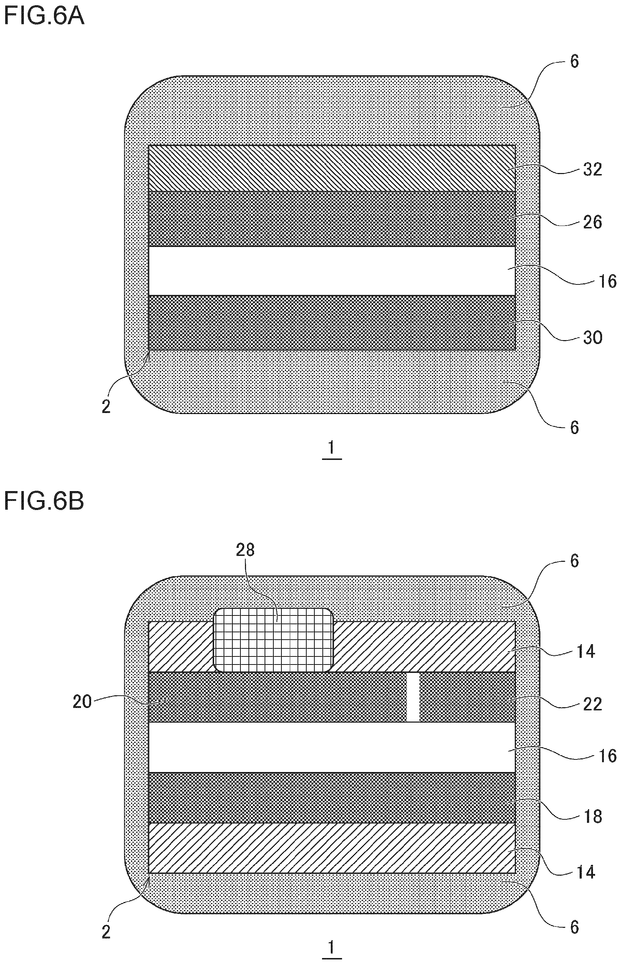

Fig. 5 is a cross-sectional view along the A-A line shown in Fig. 1A. In Fig. 5, only the sensing unit 2 is illustrated. Fig. 6A is a cross-sectional view along the B-B line shown in Fig. 5. Fig. 6B is a cross-sectional view along the C-C line shown in Fig. 5.

As shown in Figs. 5 and 6A, the insulating substrate 16 is arranged in the center of the leading edge region of the sensing unit 2. The working electrode 26 and the reagent layer 32 are stacked in this order on the front surface side of the insulating substrate 16. Further, the counter electrode 30 is stacked on the back surface side of the insulating substrate 16. The insulating substrate 16, the working electrode 26, the reagent layer 32, and the counter electrode 30 are covered with the protective film 6.

As shown in Figs. 5 and 6B, the insulating substrate 16 is arranged in the center in the region of the sensing unit 2 more toward the terminal unit 4 than the leading edge region. The reference electrode conductor 20 and the working electrode conductor 22 are stacked on the front surface side of the insulating substrate 16, and the resist film 14 is stacked thereon. Further, the reference electrode 28 is stacked on the front surface side of the reference electrode conductor 20. Further, the conductive thin film 18 and the resist film 14 are stacked in this order on the back surface side of the insulating substrate 16. The insulating substrate 16, the reference electrode conductor 20, the working electrode conductor 22, the resist film 14 on the front surface side, the reference electrode 28, the conductive thin film 18, and the resist film 14 on the back surface side are covered with the protective film 6.

The working electrode 26, the reference electrode 28, and the counter electrode 30 constitute the electrode part 34. Therefore, the electrode part 34 of this embodiment has a three-electrode type electrode structure. The electrode part 34 may include the working electrode 26 and the counter electrode 30 and may be of a two-electrode type comprised only of the working electrode 26 and the counter electrode 30.

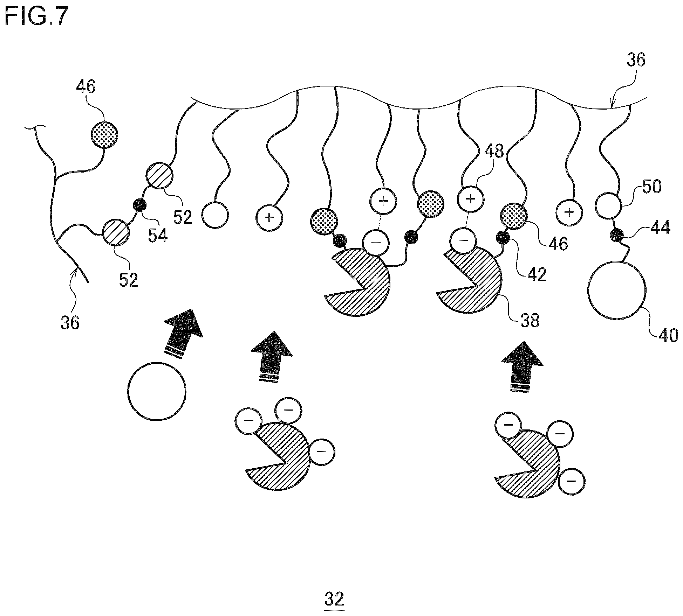

Next, the reagent layer 32 will be described in detail. Fig. 7 is a schematic diagram showing the state of a polymer 36, a protein 38, and a redox mediator 40 in the reagent layer 32. As shown in Fig. 7, the reagent layer 32 includes the polymer 36 and the protein 38. Examples of the protein 38 include oxidoreductases, antibodies (immunoglobulins), etc. In this embodiment, the protein 38 is an oxidoreductase. The reagent layer 32 of this embodiment includes the redox mediator 40 having electron transfer capability. Further, the reagent layer 32 includes a first crosslinking agent 42, a second crosslinking agent 44, and a third crosslinking agent 54. The reagent layer 32 also optionally includes other components such as conductive particles (e.g., carbon particles) and buffer solution components.

The polymer 36 includes a first binding site 46 that covalently binds to the protein 38 and a second binding site 48 that binds to the protein 38 by electrostatic interaction. Bonding between the polymer 36 and the protein 38 by covalent bonding and electrostatic interaction makes it possible to form a high molecular weight complex in which the polymer 36 and the protein 38 are tightly bonded. This inhibits the protein 38 from passing through the protective film 6 and flowing out of the sensor 1. In Fig. 7, the second binding site 48 has a positive charge, and the protein 38 has a negative charge, but the opposite may be the case.

In this embodiment, the first crosslinking agent 42 is intervened between the protein 38 and the first binding site 46 and binds both. This allows the complex to have higher molecular weight so that the outflow of the protein 38 can be further inhibited. Optionally, the protein 38 and the first binding site 46 is directly covalently bonded without the first crosslinking agent 42 intervening between them.

Further, the polymer 36 includes a third binding site 50 that covalently binds to the redox mediator 40. Covalent bonding between the polymer 36 and the redox mediator 40 makes it possible to form a high molecular weight complex in which the polymer 36 and the redox mediator 40 are bonded. This inhibits the redox mediator 40 from passing through the protective film 6 and flowing out of the sensor 1.

In this embodiment, the second crosslinking agent 44 is intervened between the redox mediator 40 and the third binding site 50 and binds both. This allows the complex to have a higher molecular weight so that the outflow of the redox mediator 40 can be further inhibited. Optionally, the redox mediator 40 and the third binding site 50 is directly covalently bound without the second crosslinking agent 44 intervening between them.

Further, bonding of the oxidoreductase as the protein 38 and the redox mediator 40 to the polymer 36 makes it possible decrease the distance between the oxidoreductase and the redox mediator 40. Thereby, the responsiveness of the reagent layer 32 can be increased. Further, the complex can have a higher molecular weight as compared to the case in which the polymer 36 binds only one of the protein 38 and the redox mediator 40. Thereby, the outflow of the protein 38 and the redox mediator 40 can be further inhibited.

The reagent layer 32 includes a plurality of polymers 36. The plurality of polymers 36 included in the reagent layer 32 may be only one type or a combination of a plurality of types. Each polymer 36 has a fourth binding site 52 that covalently binds to other polymers 36. The polymers 36 can bind to each other by covalent bonding between the fourth binding sites 52. In this embodiment, the third crosslinking agent 54 is intervened between two fourth binding sites 52 and binds both. Mutual bonding between the polymers 36 allows the complex to have an even higher molecular weight. Further, the third crosslinking agent 54 intervening between the polymers 36 to bind the polymers 36 allows the complex to have an even higher molecular weight. Thereby, the outflow of the protein 38 and the redox mediator 40 can be further inhibited. Optionally, the fourth binding sites 52 are directly covalently bonded without the third crosslinking agent 54 intervening between them.

Hereinafter, each component included in the reagent layer 32 will be described in detail.

### (Polymer 36)

The polymer 36 of this embodiment include, as structural units, the first monomer represented by the following formula (1) and the second monomer represented by the following formula (2). 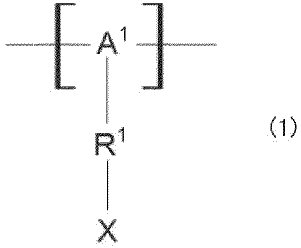 [In formula (1), A¹ is a hydrocarbon group that optionally has a substituent or includes a heteroatom in a main chain, R¹ is a hydrocarbon group that optionally has a substituent, an acyl group that optionally has a substituent, an oxyacyl group that optionally has a substituent, or a phenylene group that optionally has a substituent, X is a functional group that can be covalently bonded to the protein 38, and, when there are a plurality of first monomers, A¹, R¹ and X are the same or different] 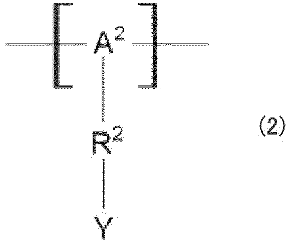 [In formula (2), A² is a hydrocarbon group that optionally has a substituent or includes a heteroatom in a main chain, and R² is a hydrocarbon group that optionally has a substituent, an acyl group that optionally has a substituent, an oxyacyl group that optionally has a substituent, or a phenylene group that optionally has a substituent, Y is a functional group that can bind to the protein 38 by electrostatic interaction, and, when there are a plurality of second monomers, A², R² and Y are the same or different]

The heteroatoms that A¹ and A² can include in the main chain are independently exemplified by oxygen (O), nitrogen (N), and silicon (Si). Examples of A¹ include -CH₂-CHR¹-, -CH₂-C(CH₃)R¹-, and the like. Examples of A² include CH₂-CHR²-, -CH₂-C(CH₃)R²-, and the like. Examples of R¹ and R² are independently exemplified by -CH₂-, -C(=O)O-CH₂-CH₂-, - C₆H₅-CH₂-, and the like. -C₅H₅- is a benzene ring.

The functional group X is preferably a group derived from at least one selected from the group consisting of amine, alcohol, phenol, thiol, carboxylic acid, hydrazine, alkoxyamine, hydroxyamine, imidazole, pyridine, acyl azide, acyl halide, alkyl halide, acid anhydride, aldehyde, ketone, alkyl sulfonate, maleimide, epoxide, aziridine, isocyanate, isothiocyanate, imidoester, sulfonyl halide, and activated ester. Examples of activated ester include succinimidyl ester, benzotriazole ester or aryl ester replaced with an electron withdrawing group including a sulfo group, a nitro group or a halo group, or a carboxylic acid ester having a carbodiimide group.

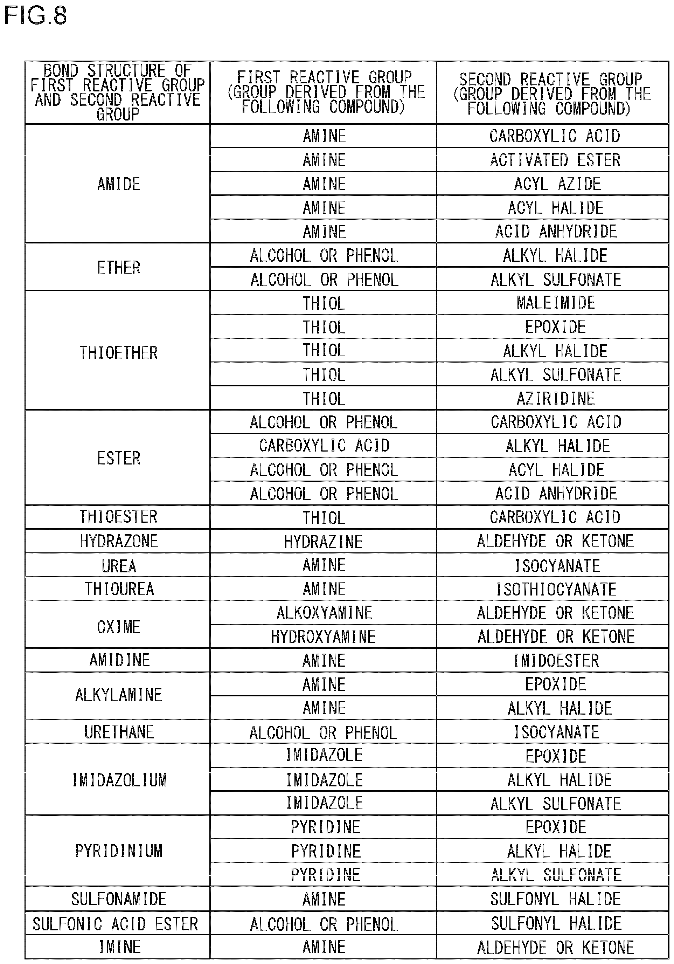

At least some functional groups X constitute the first binding site 46. In this case, the type of functional group X can be appropriately selected according to the type of the protein 38 bonded. Fig. 8 is a diagram showing combinations of reactive groups that can be covalently bonded. The first binding site 46 and the protein 38 can be covalently bonded to each other provided that one of them has the first reactive group shown in Fig. 8 and the other has the second reactive group shown in Fig. 8. Further, when the first crosslinking agent 42 is intervened between the first binding site 46 and the protein 38, one of the first crosslinking agent 42 and the first binding site 46 optionally has the first reactive group and the other optionally has the second reactive group, and one of the first crosslinking agent 42 and the protein 38 optionally has the first reactive group and the other optionally has the second reactive group.

Preferably, the functional group X is a group derived from at least one selected from the group consisting of amine, thiol, carboxylic acid, imidazole and pyridine. This makes it possible to select a more readily available crosslinking agent as the first crosslinking agent 42 so that covalent bonding between the polymer 36 and the protein 38 via the first crosslinking agent 42 can be more easily realized. It also makes reaction conditions such as temperature and pH more moderate in the crosslinking reaction between the polymer 36 and the protein 38 with or without the intervention of the first crosslinking agent 42. Thereby, deactivation of protein 38 can be inhibited.

Further, at least some functional groups X constitute the third binding site 50. In this case, the type of functional group X can be appropriately selected according to the type of redox mediator 40 bonded. That is, the third binding site 50 and the redox mediator 40 can be covalently bonded to each other provided that one of them has the first reactive group and the other has the second reactive group. Further, when the second crosslinking agent 44 intervenes between the third binding site 50 and the redox mediator 40, one of the second crosslinking agent 44 and the third binding site 50 optionally has the first reactive group and the other optionally has the second reactive group, and one of the second crosslinking agent 44 and the redox mediator 40 optionally has the first reactive group and the other optionally has the second reactive group.

The first binding site 46 and the third binding site 50 preferably have the same functional group. That is, the same type of functional group X preferably constitutes both the first binding site 46 and the third binding site 50. Thereby, covalent bonding with the protein 38 and covalent bonding with the redox mediator 40 can be realized with one type of first monomer. Accordingly, the types of monomers constituting the polymer 36 can be reduced, and the synthesis method of the polymer 36 can be simplified.

Further, at least some functional groups X constitute the fourth binding site 52. In this case, the type of functional group X can be appropriately selected according to the type of the fourth binding site 52 of the other polymer 36 that is the bonded. That is, when one of two polymers 36 has the first reactive group and the other has the second reactive group at the fourth binding site 52, they can be covalently bonded to each other. Further, when the third crosslinking agent 54 intervenes between two fourth binding sites 52, the condition for combination of the first reactive group and the second reactive group need only be met between the third crosslinking agent 54 and each fourth binding site 52.

At least two of the first binding site 46, the third binding site 50, and the fourth binding site 52 preferably have the same functional group, and more preferably, three of them have the same functional group. That is, the same type of functional group X preferably constitutes at least two of the first binding site 46, the third binding site 50, and the fourth binding site 52. Thereby, covalent bonding with the protein 38, covalent bonding with the redox mediator 40, and covalent bonding with other polymers 36 can be realized with fewer types of first monomers. Therefore, the types of monomers constituting the polymer 36 can be reduced, and the synthesis method of the polymer 36 can be simplified.

The functional group Y is preferably a cationic group derived from at least one selected from the group consisting of quaternary ammonium, quaternary phosphonium, tertiary sulfonium, and aromatic cation, or is an anion group derived from at least one selected from the group consisting of carboxylic acid, phosphonic acid, and sulfonic acid.

Quaternary ammonium has a structure in which four organic subconditions R², R³, R⁴, R⁵ are bonded to a nitrogen atom. Quaternary phosphonium has a structure in which four organic subconditions R², R³, R⁴, R⁵ are bonded to a phosphorus atom. Tertiary sulfonium has a structure in which three organic subconditions R², R³, R⁴ are bonded to a sulfur atom. R² is as described above. Optionally, R³ to R⁵ is, for example, a straight-chain or branched-chain saturated or unsaturated hydrocarbon group having 1-6 carbon atoms, which optionally has a substituent, an acyl group which optionally has a substituent, an alkoxy group which optionally has a substituent, or a phenyl group which optionally has a substituent. R³ to R⁵ are the same or different.

Examples of aromatic cations include imidazolium, oxazolium, pyrazinium, pyrazolium, pyridazinium, pyrrolidinium, pyridinium, piperidinium, thiazolium and triazolium.

The functional group Y constitutes the second binding site 48. The type of functional group Y can be appropriately selected according to the type of the protein 38 bonded. The second binding site 48 and the protein 38 can bind to each other by electrostatic interaction provided that they have opposite charges. The functional group Y by way of one example is a group different from the functional group X. Preferably, the functional group Y is a cationic group. When the protein 38 is an oxidoreductase, it should be noted that many common oxidoreductases have a negative charge under conditions near neutrality in which the sensor 1 is mainly used. For this reason, bonding by electrostatic interaction between the polymer 36 and the protein 38 can be more easily realized by configuring the functional group Y as a cationic group.

Specific examples of the polymer 36 include those shown in the following formula (3) to formula (7). m and n in formula (3) to formula (6) and l, m and n in formula (7) represent molar ratios of the respective monomers. The molar ratio of each monomer can be set as appropriate. Further, Cl⁻ is shown as an example of the counter ion in the formula (3) to formula (7).

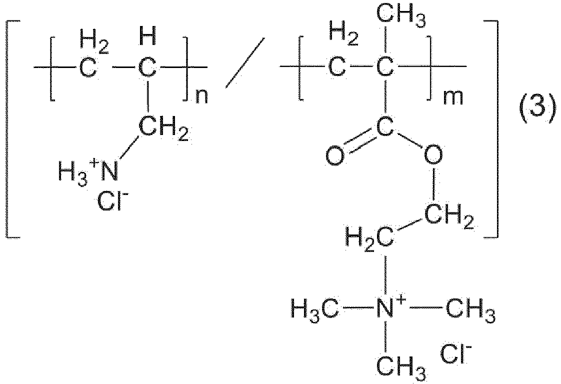

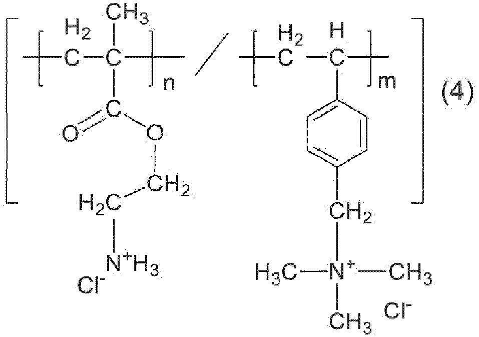

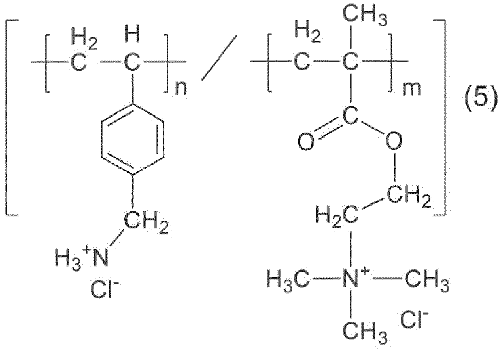

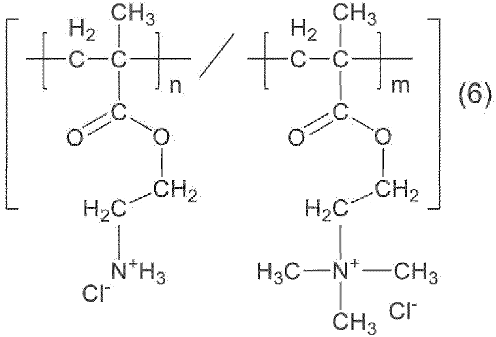

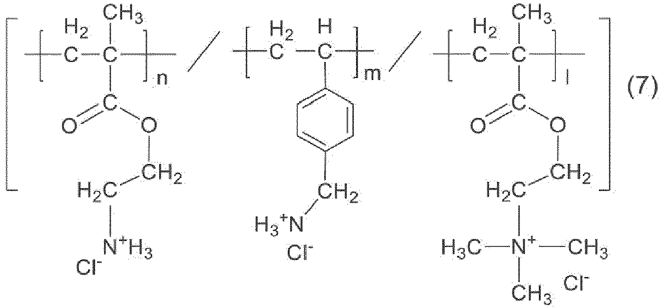

### (Protein 38)

The protein 38 binds to the first binding site 46 and the second binding site 48 of the polymer 36 and also acts on the analyte. As described above, examples of the protein 38 include oxidoreductases, antibodies (immunoglobulins), and the like. The oxidoreductase in this embodiment refers to an enzyme capable of oxidizing or dehydrogenating the analyte. Oxidoreductases are exemplified glucose oxidase, lactate oxidase, pyruvate oxidase, cholesterol oxidase, amino acid oxidase, glutamate oxidase, fructosyl amino acid oxidase, alcohol oxidase, ascorbate oxidase, fructosyl peptide oxidase, glucose dehydrogenase, lactate dehydrogenase, pyruvate dehydrogenase, amino acid dehydrogenase, glutamate dehydrogenase, 3-hydroxybutyric acid dehydrogenase, alcohol dehydrogenase and the like. A plurality of types of oxidoreductases may be combined as necessary.

In the case the protein 38 is an oxidoreductase, an amino group included in the oxidoreductase is typically covalently bonded to the first binding site 46 or the first crosslinking agent 42. Further, a thiol group, a carboxyl group, or the like included in the oxidoreductase is optionally covalently bonded to the first binding site 46 or the first crosslinking agent 42. The blending amount of the oxidoreductase is, for example, preferably 0.01 to 100 U (unit), and, more preferably, 0.05 to 50 U, and, still more preferably, 0.1 to 10 U per sensor or per measurement.

Further, the protein 38 preferably has an isoelectric point equal to or less than the pH of the usage environment when the site binding to the second binding site 48 has a positive charge. Further, when the site binding to the second binding site 48 has a negative charge, the isoelectric point of the protein 38 is preferably equal to or higher than the pH of the usage environment. The usage environment of the protein 38 refers at least to an environment in the reagent solution. Preferably, the usage environment of the protein 38 refers to both an environment in the reagent solution and an environment in the sample to be measured. By way of one example, when the site binding to the second binding site 48 has a positive charge, the isoelectric point of the protein 38 is preferably pH 9 or less, and, more preferably pH 7.4 or less, and, still more preferably, pH 5.5 or less. Further, when the site binding to the second binding site 48 has a negative charge, the isoelectric point of the protein 38 is preferably pH 5.5 or higher, and, more preferably pH 7.4 or higher, and, still more preferably, pH 9 or higher.

The oxidoreductase as the protein 38 is preferably a coenzyme-bound enzyme. Examples of coenzymes include pyrroloquinoline quinone (PQQ), nicotinamide adenine dinucleotide (NAD), nicotinamide adenine dinucleotide phosphate (NADP), flavin adenine dinucleotide (FAD), flavin mononucleotide (FMN), thiamine pyrophosphate (TPP), and coenzyme A (CoA).

When the analyte is glucose, for example, the oxidoreductase is glucose oxidase or glucose dehydrogenase (GDH). Examples of coenzyme-bound glucose dehydrogenase include pyrroloquinoline quinone (PQQ)-bound GDH and flavin adenine nucleotide (FAD)-bound GDH. Further, considering the influence of maltose in vivo, GDH preferably has an enzyme activity of 5% or less for maltose, and, more preferably, 3% or less when the enzyme activity for glucose is defined to be 100%. Examples of enzymes having such enzyme activity include FAD-bound GDH. Examples of such FAD-bound GDH include those derived from the genus Aspergillus (oryze and teleus) and those derived from the genus Mucor.

### (Redox Mediator 40)

The redox mediator 40 binds to the third binding site 50 of the polymer 36 and can reversibly become an oxidizer and a reductant to mediate transfer of electrons between substances. The redox mediator 40 is exemplified by metal complexes such as osmium complexes, ruthenium complexes, and iron complexes; quinone compounds such as benzoquinone, naphthoquinone, phenanthrenequinone, phenanthroline quinone, anthraquinone, and derivatives thereof; phenazine compounds; viologenic compounds; phenothiazine compounds; phenolic compounds, and the like. A plurality of types are optionally combined as necessary produce the redox mediator 40.

Specific examples of the redox mediator 40 include derivatives of one or more compounds selected from the group consisting of potassium ferricyanide; hexaammineruthenium; ferrocene; hydroquinone; 2-methyl-1,4-benzoquinone; 1,2-naphthoquinone-4-sulfonate; 9,10-phenanthrenequinone-2-sulfonate; 9,10-phenanthrenequinone-2,7-disulfonate; 1,10-phenanthroline-5,6-dione; anthraquinone-2-sulfonate; phenazine derivatives such as 1-methoxy-5-methylphenazinium methylsulfate and 1-methoxy-5-ethylphenazinium ethylsulfate; methylviologen; benzyl viologen; methylene green; 2-aminophenol; 2-amino-4-methylphenol; and 2,4-diaminophenol, wherein the derivative has a functional group (the first reactive group or the second reactive group shown in Fig. 8) capable of binding to the polymer 36. Examples of the above salts (sulfonates, disulfonates) include sodium salt, potassium salt, calcium salt, magnesium salt, lithium salt, and the like.

The blending amount of the redox mediator 40 is, for example, preferably 0.1 pmol to 1000 umol, and, more preferably, 5 pmol to 250 pmol, and, still more preferably, 100 pmol to 50 pmol per sensor or per measurement.

The redox mediator 40 has a site binding to the third binding site 50 or the second crosslinking agent 44. As described above, the binding site of the redox mediator 40 includes, of the first reactive group and the second reactive group, the reactive group to which the reactive group included in the third binding site 50 or the second crosslinking agent 44 is bonded.

### (First crosslinking agent 42, second crosslinking agent 44, and third crosslinking agent 54)

The first crosslinking agent 42, the second crosslinking agent 44, and the third crosslinking agent 54 is exemplified by one or more selected from the group consisting of glutaraldehyde; compounds having NHS esters at both ends such as Disuccinimidyl Succinate, Bis(3-sulfo-N-succinimidyl) Suberate Disodium Salt, 4,7,10,13,16-pentaoxanonadecanedioic acid di(N-succinimidyl) (Bis(NHS)PEG5); compounds having epoxy groups at both ends such as Poly(ethylene glycol) diglycidyl ether, Poly(propylene glycol) diglycidyl ether, 1,4-Butanediol diglycidyl ether; compounds having maleimide groups at both ends such as 1,2-Bis(maleimido) ethane), 1,11-bismaleimidotriethyleneglycol; compounds having amino groups at both ends such as 1,2-Bis(2-aminoethoxy) ethane; compounds of functional groups terminated differently at the ends such as maleimide-PEG2-succinimidyl (Maleimide-PEG2-NHS Ester) and Amino-PEG6-carboxylic Acid; and compounds having a branched chain such as 4arm-PEG10K-Succinimidyl Glutarate, 4arm-PEG10K-Maleimide, 8arm-PEG10K-Succinimidyl Succinate.

When the reagent layer 32 includes the first crosslinking agent 42 and the second crosslinking agent 44, the first crosslinking agent 42 and the second crosslinking agent 44 are preferably the same type of crosslinking agent. Further, when the reagent layer 32 includes the first crosslinking agent 42 and the third crosslinking agent 54, the first crosslinking agent 42 and the third crosslinking agent 54 are preferably the same type of crosslinking agent. Further, when the reagent layer 32 includes the second crosslinking agent 44 and the third crosslinking agent 54, the second crosslinking agent 44 and the third crosslinking agent 54 are preferably the same type of crosslinking agent. Further, when the reagent layer 32 includes the first crosslinking agent 42, the second crosslinking agent 44, and the third crosslinking agent 54, at least two of the first crosslinking agent 42, the second crosslinking agent 44, and the third crosslinking agent 54 are preferably the same crosslinking agent, and, more preferably, three of them are the same crosslinking agent. This reduces the types of crosslinking agents and so can simplify the preparation method of the reagent layer 32.

The reagent layer 32 by way of one example includes: the polymer 36 having the first binding site 46, the second binding site 48, the third binding site 50, and the fourth binding site 52; the protein 38; the redox mediator 40; the first crosslinking agent 42, and a third crosslinking agent 54. At least two of the first binding site 46, the third binding site 50, and the fourth binding site 52 preferably have the same functional group. The protein 38 binds to the first binding site 46 via the first crosslinking agent 42. Further, the protein 38 binds to the second binding site 48. The redox mediator 40 binds directly to the third binding site 50 without the second crosslinking agent 44 intervening between them. The fourth binding sites 52 of the two polymers 36 binds to each other via the third crosslinking agent 54. The first crosslinking agent 42 and the third crosslinking agent 54 are the same crosslinking agent.

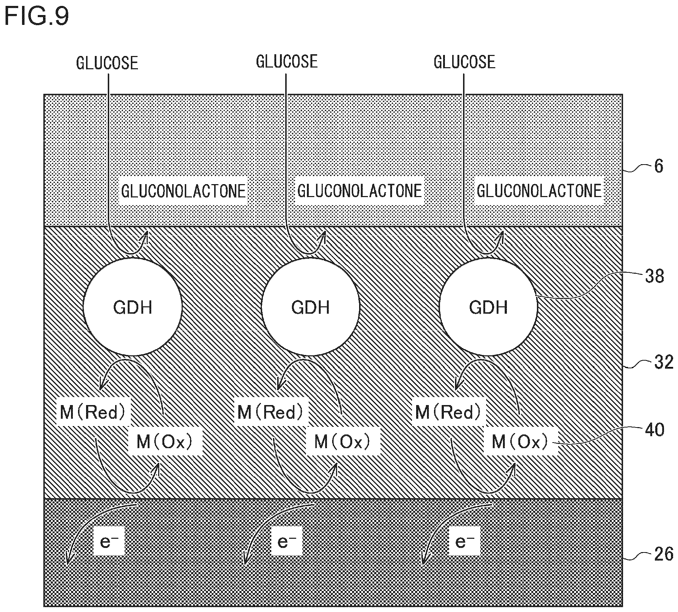

Next, the principle of measuring the analyte by the sensor 1 will be described. Fig. 9 is a schematic diagram for explaining the measurement principle of the sensor 1. By way of example, a description will be given of a case where the sensor 1 is an electrochemical sensor, the analyte is glucose, and the protein 38 is glucose dehydrogenase (GDH).

First, glucose in the blood or subcutaneous interstitial fluid permeates the protective film 6 and reaches the reagent layer 32. Next, glucose is oxidized by glucose dehydrogenase to produce gluconolactone. At the same time, the redox mediator 40 is reduced, and the oxidant (M(Ox)) becomes a reductant (M(Red)). Then, the reductant of the redox mediator 40 is oxidized on the surface of the working electrode 26, the reductant (M(Red)) becomes an oxidant (M(Ox)), and electrons are generated at the same time. These electrons are delivered to the working electrode 26 and are measured as a current value.

As described above, the polymer 36 according to this embodiment binds the protein 38 by covalent bonding by the first binding site 46 and electrostatic interaction by the second binding site 48. Thereby, the outflow of the protein 38 outside the sensor can be inhibited. This further inhibits a decrease in the durability and measurement sensitivity of the sensor 1. In addition, negative impacts on the living body, etc. can be further inhibited. Further, the polymer 36 binds the redox mediator 40 by covalent bonding by the third binding site 50. Thereby, the outflow of the redox mediator 40 outside the sensor can be suppressed. This further inhibits a decrease in the durability and measurement sensitivity of the sensor 1 and further inhibits negative impacts on the living body, etc. Further, the polymer 36 also binds to other polymers 36 by covalent bonding by the fourth binding site 52. This further inhibits a decrease in the durability and measurement sensitivity of the sensor 1 and further inhibits negative impacts on the living body, etc.

Further, the first crosslinking agent 42 intervenes between the protein 38 and the first binding site 46. Also, the second crosslinking agent 44 intervenes between the redox mediator 40 and the third binding site 50. Further, the third crosslinking agent 54 intervenes between the two fourth binding sites 52. These allow the complex of the polymer 36, the protein 38 and the redox mediator 40 to have a higher molecular weight. Thereby, the outflow of the protein 38 and the redox mediator 40 outside the sensor can be further inhibited.

The embodiments of the present disclosure are described above in detail. The embodiments described above are merely specific examples of practicing the present disclosure. The details of the embodiment shall not be construed as limiting the technical scope of the present disclosure. A number of design modifications such as modification, addition, deletion, etc. of constituting elements may be made to the extent that they do not depart from the idea of the invention defined by the claims. New embodiments with design modifications will provide the combined advantages of the embodiment and the variation. Although the details subject to such design modification are emphasized in the embodiment described above by using phrases such as "of this embodiment" and "in this embodiment", details not referred to as such are also subject to design modification. Any combination of the above constituting elements is also useful as a mode of the present disclosure. Hatching in the cross section in the drawings should not be construed as limiting the material of the hatched object.

The embodiment may be defined by the following items.

### [Item 1]

A polymer (36) included in a reagent layer (32) of a sensor (1) for measuring an analyte in a sample, including:
a first binding site (46) covalently bonded to a protein (38) acting on the analyte; and
a second binding site (48) that binds to the protein (38) by electrostatic interaction.

### [Item 2]

The polymer (36) according to item 1, including, as structural units:
a first monomer represented by the above formula (1); and
a second monomer represented by the above formula (2).

### [Item 3]

The polymer (36) according to item 2,
wherein the functional group X is a group derived from at least one selected from the group consisting of amine, alcohol, phenol, thiols, carboxylic acid, hydrazine, alkoxyamine, hydroxyamine, imidazole, pyridine, acyl azide, acyl halide, alkyl halide, acid anhydride, aldehyde, ketone, alkyl sulfonate, maleimide, epoxide, aziridine, isocyanate, isothiocyanate, imidoester, sulfonyl halide, and activated ester, and
wherein the functional group Y is a cationic group derived from at least one selected from the group consisting of quaternary ammonium, quaternary phosphonium, tertiary sulfonium, and aromatic cation, or is an anion group derived from at least one selected from the group consisting of carboxylic acid, phosphonic acid, and sulfonic acid.

### [Item 4]

The polymer (36) according to any one of items 1 to 3,
wherein the protein (38) is an oxidoreductase.

### [Item 5]

The polymer (36) according to item 4,
wherein the polymer (36) includes a third binding site (50) that covalently binds to a redox mediator (40) having electron transfer capability.

### [Item 6]

The polymer (36) according to item 5,
wherein the first binding site (46) and the third binding site (50) have the same functional group.

### [Item 7]

A reagent layer (32) of a sensor (1) for measuring an analyte in a sample, including:
the polymer (36) according to any one of items 1 through 6; and
the protein (38) that binds to the polymer (36) and also acts on the analyte.

### [Item 8]

The reagent layer (32) according to item 7, including:
a first crosslinking agent (42) that binds the protein (38) and the first binding site (46).

### [Item 9]

The reagent layer (32) according to item 7 or 8,
wherein the protein (38) is an oxidoreductase, and
wherein the polymer (36) includes a third binding site (50) that covalently binds to a redox mediator (40) having electron transfer capability, and
wherein the reagent layer (32) includes the redox mediator (40) that binds to the polymer (36).

### [Item 10]

The reagent layer (32) according to item 9, including:
a second crosslinking agent (44) that binds the redox mediator (40) and the third binding site (50).

### [Item 11]

The reagent layer (32) according to item 10, including:
a first crosslinking agent (42) that binds the oxidoreductase (38) and the first binding site (46),
wherein the first crosslinking agent (42) and the second crosslinking agent (44) are the same crosslinking agent.

### [Item 12]

The reagent layer (32) according to any one of items 7 through 11,
wherein the reagent layer (32) includes a plurality of polymers (36),
wherein each polymer (36) has a fourth binding site (52) that covalently binds to other polymers (36), and
wherein the reagent layer (32) includes a third crosslinking agent (54) that binds the fourth binding sites (52) to each other.

### [Item 13]

The reagent layer (32) according to item 12,
wherein the protein (38) is an oxidoreductase, and
wherein the polymer (36) includes a third binding site (50) that covalently binds to a redox mediator (40) having electron transfer capability, and
wherein at least two of the first binding site (46), the third binding site (50), and the fourth binding site (52) have the same functional group.

### [Item 14]

The reagent layer (32) according to item 12 or 13,
wherein the protein (38) is an oxidoreductase,
wherein the polymer (36) includes a third binding site (50) that covalently binds to a redox mediator (40) having electron transfer capability,
wherein the reagent layer (32) includes a first crosslinking agent (42) that binds the oxidoreductase (38) and the first binding site (46), and a second crosslinking agent (44) that binds the redox mediator (40) and the third binding site (50), and
wherein at least two of the first crosslinking agent (42), the second crosslinking agent (44), and the third crosslinking agent (54) are the same crosslinking agent.

### [Item 15]

A sensor (1) including:
an electrode part (34) that includes a working electrode (26) and a counter electrode (30); and
the reagent layer (32) according to any one of items 7 through 14 arranged so as to be in contact with the working electrode (26).

### [Item 16]

The sensor (1) according to item 15, including:
a protective layer (6) that covers the reagent layer (32) .

### [Exemplary embodiments]

Hereinafter, exemplary embodiments of the present invention will be described, but these exemplary embodiments are merely examples for suitably describing the present invention and shall not be interpreted as limiting the present invention in any way.

### (Synthesis of polymer A)

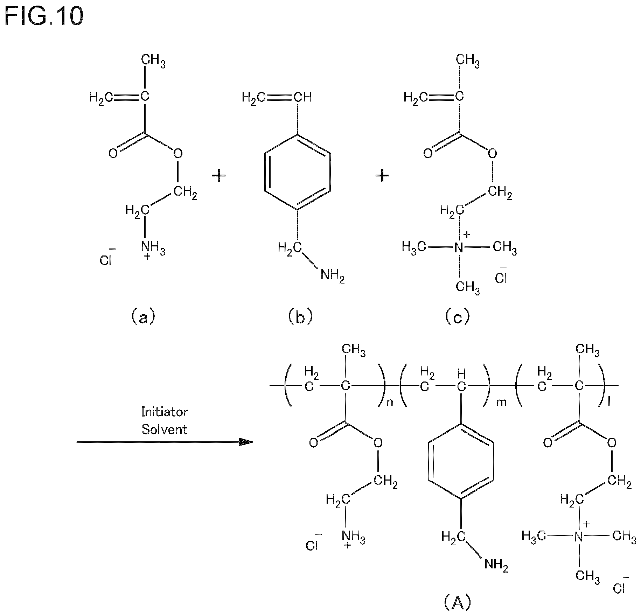

Fig. 10 is a diagram showing the polymerization reaction of polymer A. First, 2-aminoethyl methacrylate hydrochloride (monomer a), (4-vinylphenyl) methanamine (monomer b), and methachlorylcholine chloride (monomer c) were prepared. Then, 0.97 mmol of monomer a, 0.97 mmol of monomer b, 11.97 mmol of monomer c, 0.08 mmol of 2,2'-azobis (2-methylpropionamidine) dihydrochloride (hereinafter referred to as V-50), and 227 mmol of ethanol were added to a four-neck flask.

Next, a stirrer tip, a thermometer, and a decompression/Ar gas line were set in the four-neck flask. The sample in the flask was stirred at room temperature and was degassed by repeating decompression in the four-neck flask and supply of Ar gas 10 times by using the decompression/Ar gas line. After the degassing process, the four-neck flask was sealed, and the polymerization reaction shown in Fig. 10 was performed at a reaction temperature of 60°C for 24 hours. The progress and termination of polymerization were confirmed by measuring the loss of monomers by ¹H-NMR. After completion of the reaction, the reaction solution was placed in a dialysis membrane (MWCO: 3500) and dialyzed for 3 days. At that time, the solution was changed twice a day. After completion of the dialysis, the obtained reactant was lyophilized to obtain polymer A. Polymer A corresponds to a polymer in which n:m:l in the above formula (7) is such that n:m:l=21.2:8.1:70.7. The molar ratio of each monomer was calculated from the integral ratio of a signal in the NMR spectrum. The same applies to the following polymers B, C, E, F. Further, polymer A was found to have a number average molecular weight Mn of 89642 and a weight average molecular weight Mw of 502171 as a result of measurement by gel penetration chromatography (GPC).

### (Synthesis of polymer B)

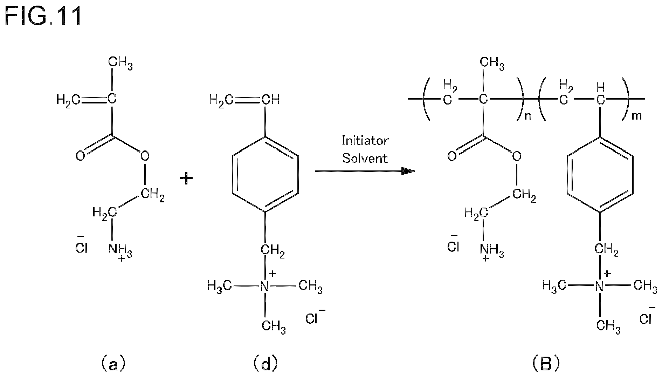

Fig. 11 is a diagram showing the polymerization reaction of polymer B. First, monomer a and (vinylbenzyl) trimethylammonium chloride (monomer d) were prepared. Then, 7.36 mmol of monomer a, 7.36 mmol of monomer d, 0.07 mmol of V-50, and 616 mmol of ion exchange water were added to a four-neck flask. Subsequently, the same process as that of polymer A was performed, and the polymerization reaction shown in Fig. 11 was performed to obtain polymer B. Polymer B corresponds to a polymer in which n:m in the above formula (4) is such that n:m=48.5:51.5.

### (Synthesis of polymer C)

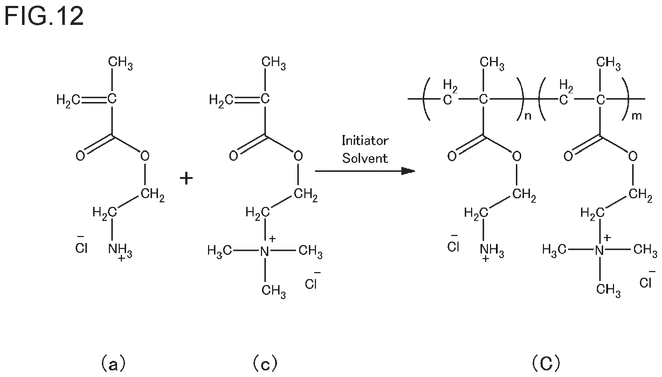

Fig. 12 is a diagram showing the polymerization reaction of polymer C. First, monomer a and monomer c were prepared. Then, 1.14 mmol of monomer a, 10.22 mmol of monomer c, 0.06 mmol of V-50, and ion exchange water were added to a four-neck flask. Subsequently, the same process as that of polymer A was performed, and the polymerization reaction shown in Fig. 12 was performed to obtain polymer C (the reaction time was appropriately adjusted as necessary). Polymer C corresponds to a polymer in which n:m in the above formula (6) is such that n:m=13.3:86.7.

### (Preparation of mediator-bonded polymer D as a comparative example)

Phenazine, and, specifically 5-{[(2,5-dioxopyridin-1-yl) oxy]-5-oxopentyl}-1-methoxyphenazinium nitrate (manufactured by Tokyo Chemical Industry Co., Ltd.), was prepared as a mediator. 0.47 mg of this mediator was measured and dissolved in 47 µL of MiliQ water. In addition, poly(L-lysine) hydrochloride (PLKC800: manufactured by Allamanda Polymers) was prepared as polymer D of the comparative example. This polymer D corresponds to a polymer that does not have the second binding site 48 that binds to glucose dehydrogenase used in the reagent preparation described later. 0.513 mg of polymer D was measured and dissolved in 51.3 µL of MiliQ water. Further, 9.59 mg of condensation agent (WSC WO01: manufactured by Dojin Chemical) was measured and dissolved in 479.3 µL of MiliQ.

These three solutions were mixed with 96 µL of 250 mM2-morpholinoethanesulfonic acid (MES) buffer solution (pH 6.0) and adjusted by MiliQ water to result in a total volume of 1200 µL. Thereafter, the reaction was allowed to progress for about 20 hours while the mixed solution was stirred at room temperature. After completion of the reaction, ultrafiltration of the reaction solution was performed several times by using a centrifugal ultrafiltration filter (Amicon Ultra-4 50k: manufactured by Merck Millipore), and the solution excluding small molecules was collected as a solution of mediator-bound polymer D (poly (L-lysine) bonded phenazine).

### (Preparation of mediator-bonded polymer A)

A solution of mediator-bonded polymer A was prepared by a method similar to that of producing mediator-bonded polymer D, except that 2.63 mg of polymer A was measured instead of polymer D and dissolved in 175.4 µL of MiliQ water.

### (Preparation of mediator-bonded polymer B)

A solution of mediator-bonded polymer B was prepared in a method similar to that of producing mediator-bonded polymer D, except that 1.56 mg of polymer B was measured instead of polymer D and dissolved in 104.1 µL of MiliQ water.

### (Preparation of mediator-binding polymer C)

A solution of mediator-bonded polymer C was prepared in a manner similar to that of mediator-bonded polymer D, except that 6.08 mg of polymer C was measured instead of polymer D and dissolved in 405.2 µL of MiliQ water.

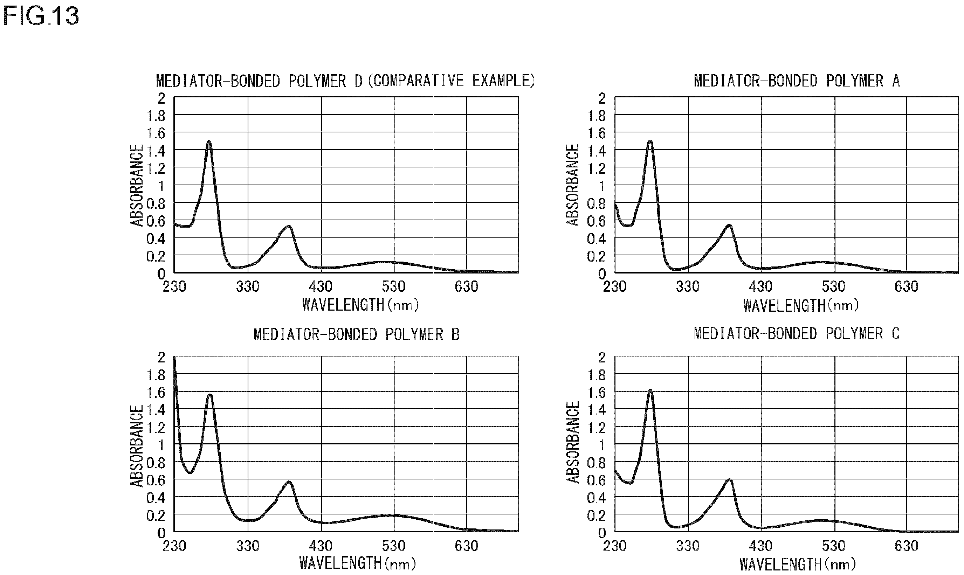

The solution of each mediator-bonded polymer was diluted 25 times, and each was added to a microplate in an amount of 100 µL. Then, the absorption spectrum of each solution was measured using a plate reader. Fig. 13 shows the results. UV-Star (registered trademark) 96Well F-Boden (manufactured by Greiner Bio One) was used as the microplate. Infinite (registered trademark) M200 Pro (manufactured by Tecan) was used as the plate reader.

Fig. 13 is a diagram showing the absorption spectrum of each mediator-bonded polymer. As shown in Fig. 13, peaks were observed between the wavelength of 230 nm to 330 nm and the wavelength of 330 nm to 430 nm in every mediator-bonded polymer. These two peaks are peaks that originate from phenazine. From this, it was confirmed that phenazine binds to each polymer.

### (Preparation of reagent solution)

The reagents shown below were mixed to result in the following final concentration. A reaction was promoted for about 1 hour to prepare the reagent solution. The carbon dispersion liquid was used after treatment with an ultrasonic bath for about 10 minutes before use.
* Sodium phosphate buffer solution (pH 6.5) Final concentration 8mM
* FAD-dependent glucose dehydrogenase (GDH GLD1:manufactured by BBI International) Final concentration 2000 U/mL
* 25% glutaraldehyde solution (manufactured by Wako Pure Chemical Industry Co., Ltd.) Final concentration 0.005% (wt/v)
* Each mediator-bonded polymer Final concentration 1 (equivalent to absorbance of 1 at 386 nm)
* Carbon dispersion liquid Final concentration 4mg/mL

The sodium phosphate buffer solution was prepared with disodium hydrogen phosphate (manufactured by Wako Pure Chemical Industry Co., Ltd.) and sodium dihydrogen phosphate (manufactured by Wako Pure Chemical Industry Co., Ltd.). The carbon dispersion liquid was obtained by mixing ketjen black (EC300J: manufactured by Lion Specialty Chemicals Co., Ltd.) with 5 mg/mL of hydroxypropyl cellulose (manufactured by NISSO HPCL Japan Soda Co., Ltd.) solution so as to result in a carbon concentration of 16 mg/mL and treating the mixture with an ultrasonic homogenizer for more than 3 minutes. The concentration of each mediator-bonded polymer in the solution was adjusted based on the concentration of each polymer in the solution checked by diluting the solution of each polymer 25 times before adding the solution to the microplate in an amount of 100 µL and measuring the absorption spectrum with a plate reader.

### (Preparation of polymer solution for protective film)

The reagents shown below were mixed to result in the following final concentration, and a polymer solution for protective film was prepared.
* Poly (ter. butyl methacrylate-b-4-vinylpyridine) (manufactured by Polymer Source, hereinafter referred to as tBuMA4VP) Final concentration 7.11%
* Random copolymer of polypropylene glycol methyl ether-styrene-4-vinylpyridine (manufactured by Polymer Source, hereinafter referred to as PGMAS4VP) Final concentration 0.89%
* Poly (ethylene glucol) dimethyl ether (manufactured by Sigma-Aldrich, hereinafter referred to as PEGDGE) Final concentration 0.98%
* HEPES buffer solution (pH 8.0) final concentration 5mM

tBuMA 4VP, PGMAS4VP and PEGDGE were used dissolved in ethanol. The number average molecular weight Mn of tBuMA 4VP is 80,000 for poly (ter. butyl methacrylate) and 77,000 for poly (4-vinylpyridine). Mw/Mn of tBuMA4VP is 1.15. PGMAS4VP is polypropylene glycol methyl ether: styrene: 4-vinylpyridine=24:16:60, the number average molecular weight Mn is 52,000, the weight average molecular weight Mw is 94,000, and Mw/Mn is 1.8. The number average molecular weight Mn of PEGDGE is up to1,000. The HEPES buffer solution was prepared by using 2-[4-(2-hydroxyethyl)-1-piperazinyl] ethanesulfonic acid (manufactured by Dojin Chemical).

### (Preparation of sensor electrodes)

A gold electrode was formed on an insulating substrate by sputtering. The reagent solutions including the respective mediator-bonded polymers were applied to different gold electrodes in an amount of 0.4 µL and dried for about 15 minutes. 0.4 µL of the reagent solution was again applied to each gold electrode and then dried overnight. Thereby, a reagent layer was formed on each gold electrode. Each gold electrode formed with the reagent layer was immersed in the polymer solution for a protective film, pulled up, and dried, which steps were repeated a plurality of times. After completion of this work, the metal electrode was dried for 1 day, and a protective film was formed on each gold electrode. Through the above steps, a sensor electrode including a reagent layer including each mediator-bonded polymer and a protective film covering the reagent layer was obtained.

### (Sensor durability evaluation)

A three-electrode electrode part was fabricated by using the sensor electrode thus fabricated as the working electrode and combining the gold electrode as a counter electrode and an Ag/AgCl electrode (saturated KCl) (manufactured by BAS) as a reference electrode. Then, the time change of electric current in a phosphate buffer physiological saline solution was measured by an amperometric method by using a potentiostat (manufactured by BAS).

Specifically, glucose was added to result in theoretical values of 50 mg/dL, 150 mg/dL, 300 mg/dL, and 500 mg/dL every 500 seconds since 1000 seconds after the start of measurement, and the current response value was continuously measured. After the measurement, the electrode was stored in a phosphate buffer physiological saline solution at 37°C. After storage for 1 day, similar measurements were made. The current values at glucose concentrations of 50 mg/dL, 150 mg/dL, and 300 mg/dL were calculated as the average of 5 measurements taken, after addition of glucose to be measured, 5 seconds before, 10 seconds before, 15 seconds before, 20 seconds before, and 25 seconds before the next addition of glucose to be measured.

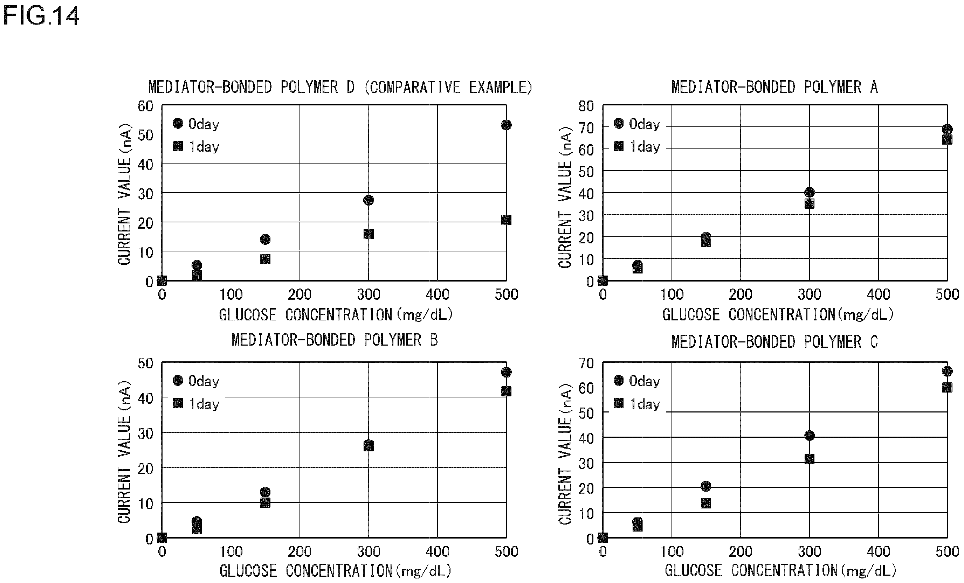

Further, the current value at the final concentration is defined as the average of 5 measurements taken 480 seconds after, 485 seconds after, 490 seconds after, 495 seconds after, and 500 seconds after the addition of a glucose solution to result in the glucose concentration of 500 mg/dL. The current value at each glucose concentration is a current value obtained after performing a background correction process that subtracts the current value at the glucose concentration of 0 mg/dL. Fig. 14 shows the results.

Fig. 14 is a diagram illustrating the results of durability evaluation of the sensor. As shown in Fig. 14, mediator-bonded polymers A to C showed smaller changes in current values between the initial day and after 1 day of storage as compared with mediator-bonded polymer D (comparative example). In particular, decrease in the current value was remarkably suppressed in the high concentration range of 300 to 500 mg/dL. From this, it was confirmed that, according to mediator-bonded polymer A to C, the outflow of oxidoreductase (protein) and the redox mediator can be inhibited, and thus the durability of the sensor can be improved.

The inventors also actually synthesized polymer E and polymer F. The synthesis procedure of each polymer will be described below.

### (Synthesis of polymer E)

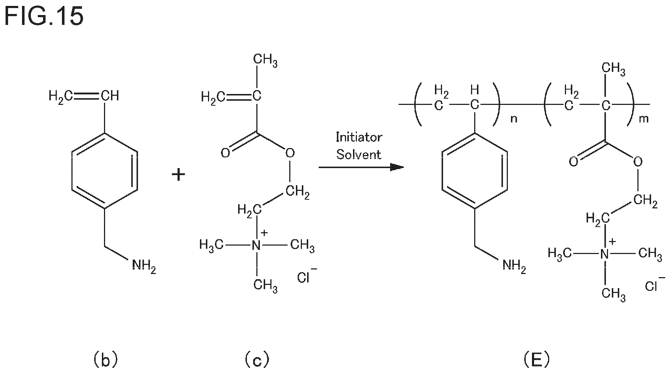

Fig. 15 is a diagram showing the polymerization reaction of polymer E. First, monomer b and monomer c were prepared. Then, 0.85 mmol of monomer b, 7.61 mmol of monomer c, and 0.25 mmol of 2,2'-azobis (4-methoxy-2,4-dimethylvaleronitrile) (hereinafter referred to as V-70) were added to a four-neck flask. Subsequently, the same process as that of polymer A was performed, and the polymerization reaction shown in Fig. 15 was performed to obtain polymer E (the reaction temperature and the reaction time were appropriately adjusted as necessary). Polymer E corresponds to a polymer in which n:m in the above formula (5) is such that n:m=15.0:85.0.

### (Synthesis of polymer F)

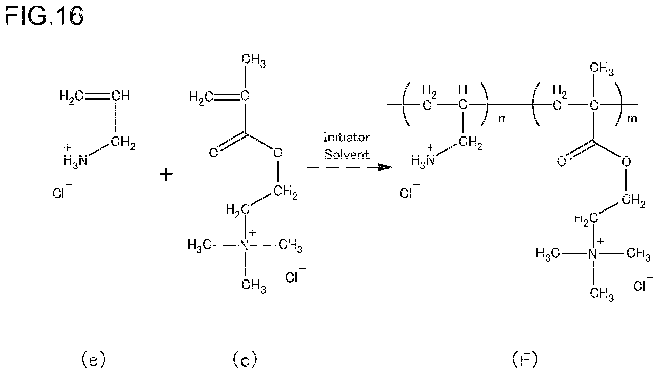

Fig. 16 is a diagram showing the polymerization reaction of polymer F. First, allylamine hydrochloride (monomer e) and monomer c were prepared. Then, 92.22 mmol of monomer e, 9.22 mmol of monomer c, 66.1 mmol of V-50, and 590 mmol of ion-exchange water were added to a four-neck flask. Subsequently, the same process as in that of polymer A was performed, and the polymerization reaction shown in Fig. 16 was performed to obtain polymer F. Polymer F corresponds to a polymer in which n:m in the above formula (3) is such that n:m=5.8:94.2.

### [INDUSTRIAL APPLICABILITY]

The present disclosure can be used in polymers, reagent layers and sensors.

### [REFERENCE SIGNS LIST]

1 sensor, 6 protective film, 26 working electrode, 30 counter electrode, 32 reagent layer, 34 electrode part, 36 polymer, 38 protein, 40 redox mediator, 42 first crosslinking agent, 44 second crosslinking agent, 46 first binding site, 48 second binding site, 50 third binding site, 52 fourth binding site, 54 third crosslinking agent

## Claims

1. A polymer included in a reagent layer of a sensor for measuring an analyte in a sample, comprising:
a first binding site covalently bonded to a protein acting on the analyte;
and
a second binding site that binds to the protein by electrostatic interaction.

2. The polymer according to claim 1, comprising, as structural units:
a first monomer represented by formula (1); and 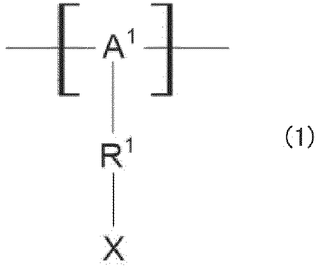 [In formula (1), A¹ is a hydrocarbon group that optionally has a substituent or includes a heteroatom in a main chain, R¹ is a hydrocarbon group that optionally has a substituent, an acyl group that optionally has a substituent, an oxyacyl group that optionally has a substituent, or a phenylene group that optionally has a substituent, X is a functional group that can be covalently bonded to the protein, and, when there are a plurality of first monomers, A¹, R¹ and X are the same or different]
a second monomer represented by formula (2) 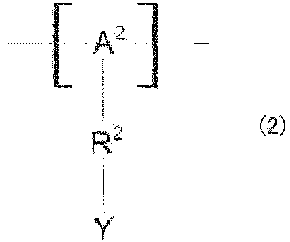 [In formula (2), A² is a hydrocarbon group that optionally has a substituent or includes a heteroatom in a main chain, and R² is a hydrocarbon group that optionally has a substituent, an acyl group that optionally has a substituent, an oxyacyl group that optionally has a substituent, or a phenylene group that optionally has a substituent, Y is a functional group that can bind to the protein by electrostatic interaction, and, when there are a plurality of second monomers, A², R² and Y are the same or different].

3. The polymer according to claim 2,
wherein the functional group X is a group derived from at least one selected from the group consisting of amine, alcohol, phenol, thiols, carboxylic acid, hydrazine, alkoxyamine, hydroxyamine, imidazole, pyridine, acyl azide, acyl halide, alkyl halide, acid anhydride, aldehyde, ketone, alkyl sulfonate, maleimide, epoxide, aziridine, isocyanate, isothiocyanate, imidoester, sulfonyl halide, and activated ester, and
wherein the functional group Y is a cationic group derived from at least one selected from the group consisting of quaternary ammonium, quaternary phosphonium, tertiary sulfonium, and aromatic cation, or is an anion group derived from at least one selected from the group consisting of carboxylic acid, phosphonic acid, and sulfonic acid.

4. The polymer according to any one of claims 1 to 3,
wherein the protein is an oxidoreductase.

5. The polymer according to claim 4,
wherein the polymer includes a third binding site that covalently binds to a redox mediator having electron transfer capability.

6. The polymer according to claim 5,
wherein the first binding site and the third binding site have the same functional group.

7. A reagent layer of a sensor for measuring an analyte in a sample, comprising:
the polymer according to any one of claims 1 through 6; and
the protein that binds to the polymer and also acts on the analyte.

8. The reagent layer according to claim 7, comprising:
a first crosslinking agent that binds the protein and the first binding site.

9. The reagent layer according to claim 7 or 8,
wherein the protein is an oxidoreductase, and
wherein the polymer includes a third binding site that covalently binds to a redox mediator having electron transfer capability, and
wherein the reagent layer includes the redox mediator that binds to the polymer.

10. The reagent layer according to claim 9, comprising:
a second crosslinking agent that binds the redox mediator and the third binding site.

11. The reagent layer according to claim 10, comprising:
a first crosslinking agent that binds the oxidoreductase and the first binding site,
wherein the first crosslinking agent and the second crosslinking agent are the same crosslinking agent.

12. The reagent layer according to any one of claims 7 through 11,
wherein the reagent layer includes a plurality of polymers,
wherein each polymer has a fourth binding site that covalently binds to other polymers, and
wherein the reagent layer includes a third crosslinking agent that binds the fourth binding sites to each other.

13. The reagent layer according to claim 12,
wherein the protein is an oxidoreductase, and
wherein the polymer includes a third binding site that covalently binds to a redox mediator having electron transfer capability, and
wherein at least two of the first binding site, the third binding site, and the fourth binding site have the same functional group.

14. The reagent layer according to claim 12 or 13,
wherein the protein is an oxidoreductase,
wherein the polymer includes a third binding site that covalently binds to a redox mediator having electron transfer capability,
wherein the reagent layer includes a first crosslinking agent that binds the oxidoreductase and the first binding site, and a second crosslinking agent that binds the redox mediator and the third binding site, and
wherein at least two of the first crosslinking agent, the second crosslinking agent, and the third crosslinking agent are the same crosslinking agent.

15. A sensor comprising:
an electrode part that includes a working electrode and a counter electrode; and
the reagent layer according to any one of claims 7 through 14 arranged so as to be in contact with the working electrode.

16. The sensor according to claim 15, comprising:
a protective layer that covers the reagent layer.
